# EUROPEAN PATENT APPLICATION

(11) **EP 1 310 562 A1**
(43) Date of publication of application: **14.05.2003**
(21) Application number: 01960746.4
(22) Date of filing: 02.08.2001
(51) Int. Cl.: C12P 7/22, A23L 1/30

(54) **ENZYMATIC SYNTHESIS OF ANTIOXIDANT HYDROXYTYROSOL**

(30) Priority: 11.08.2000 ES 200002073
(71) Applicant: CONSEJO SUPERIOR DE INVESTIGACIONES CIENTIFICAS, 28006 Madrid (ES)
(72) Inventor: Espin de Gea, Juan Carlos Ctro. E. y B.A. del Seg., 30080 Murcia (ES); De Tomas Barberan, Francisco A. C.E.y B.A.del Seg., 30080 Murcia (ES); Garcia Viguera, Maria C. Ctro. E. y B.A. del Seg., 30080 Murcia (ES); Ferreres de Arce, Federico Ctro. E. y B.A. del Seg, 300080 Murcia (ES); Soler Rivas, Cristina, 6700 Wageningen (NL); Wichers, Harry J., 6700 Wageningen (NL)
(74) Representative: Ungria Lopez, Javier
(86) International application number: ES0100314
(87) International publication number: WO02016628

(57) **Abstract**

The reaction occurs in a medium buffered with phosphate in an aqueous medium, at neutral pH and at room temperature. Hence, the reaction medium consists of: tyrosol, as precursor; (commercial) mushroom tyrosinase to catalyze the process and vitamin C in excess. Reaction starts after initial shaking. The reaction stops when the concentration of the initial tyrosol is exhausted. In order for the reaction to continue, it will suffice to add more tyrosol (always ensuring that the vitamin C/tyrosol ratio is more than 1). Once the desired concentration of hydroxytyrosol is obtained, it is filtered. The enzyme with molecular weight over twice the size of the pore is retained in the filter and can be used once again. This first extract, which is very enriched in antioxidant hydroxytyrosol with a high antioxidant capacity (in the present case, in combination with vitamin C), can already be used as a food additive.

## Description

### 2. TECHNICAL FIELD

Field: Food Technology. Chemical-Pharmaceutical Field.

Use: Food industry (addition of natural antioxidants in the production of juices, soups, baby foods, etc.) Chemical and pharmaceutical industries. Method to obtain a commercially unavailable compound.

### 3. PRIOR ART

There is evidence about the benefits provided by including virgin olive oil in our diet. The beneficial effect provides protection against cardiovascular diseases and certain types of cancer (Assmann et al., 1997). A suitable profile of fatty acids and some minor compounds, in particular, phenolic compounds, are responsible for this effect. These substances inhibit the production of free radicals due to their antioxidant properties, providing the oil with stability against oxidation of lipids. Hence, virgin olive oil has extraordinary resistance against becoming rancid, as well as a high antiradical capacity (Espin et al., 2000a). Hydroxytyrosol, which has been the object of studies where its numerous beneficial properties for health have been emphasized (Visioli and Galli, 1995, 1998; Visioli et al., 1999; Arouma et al., 1998), stands out among these phenolic compounds. The overall effect that this compound provides coincides with widespread consumption of olive oil, in other words, the protection against cardiovascular diseases and certain types of cancer. The lower incidence of these pathologies is characteristic in areas with a traditional Mediterranean diet, combined with the consumption of this oil. Very recently the good bioavailability of hydroxytyrosol justifying its inclusion in the diet contributes decisively to the above-mentioned beneficial effect, has been established (Manna et al., 2000; Visioli et al., 2000).

Hydroxytyrosol is commercially unavailable. No business firm currently supplies it. Therefore, researchers have to synthesize it chemically or else, extract it from olive oil itself or from the waste waters used in the production process of this oil.

There are various chemical synthesis protocols (Schöpf et al., 1949; Baraldi et al., 1983; Bianco et al., 1988; Verhe et al., 1992; Garcia et al., 1996; Capasso et al., 1999). Obviously, the most recent ones are the most optimized methods and they are disclosed hereinafter.

The Garcia et al. protocol (1996) consists of obtaining hydroxytyrosol by alkaline hydrolysis of oleuropein (an ester that contains hydroxytyrosol, and that is found in olives and in a smaller amount, in oil). This method uses 6 M sodium hydroxide, 37% hydrochloric acid and active carbon. This process has several disadvantages:
* Oleuropein is difficult to find commercially (it is not supplied by the large business firms: Sigma, Aldrich, Fluka, Merck, Across, etc.) It is a relatively expensive reagent.
* Reproduced in the laboratory, the yield of the process is variable (at times yields of 80% are obtained, and other times, the amount of hydroxytyrosol is insignificant.) Therefore, reproducibility is very limited.
* The protocol involves almost 1 day of work (preparation of reagents, bubbling in nitrogen for 5 hours, applying different steps and the final control of the process). Hydroxytyrosol, when it is obtained, is finally in a 0.1 M hydrochloric acid solution.
* Furthermore, it considers the use of highly toxic-inflammable-irritant reagents (sodium hydroxide, hydrochloric acid, diethyl ether), which implies extreme care in the preparation and the risk of the presence of traces of these compounds in the final hydroxytyrosol synthesized in this way.

The Capasso et al. protocol (1999) considers the use of 3,4-dihydroxyphenylacetic acid as a precursor. Starting with this, hydroxytyrosol is synthesized with the use of lithium hydride and aluminum in the solvent tetrahydrofuran. Other reagents used are the solvent ethyl acetate, hydrochloric acid, sodium bicarbonate and sodium sulfate. The global yield tends to be 80%. However, it has serious inconveniences.
* The precursor, 3,4-dihydroxyphenylacetic acid, costs 4530 pesetas per gram (Sigma-Aldrich). As it will be seen hereinafter, this increases the cost of the process in comparison with the alternative method proposed in this specification.
* This protocol also considers the use of toxic and/or contaminating reagents, some described in the above protocol. The solvents tetrahydrofuran and ethyl acetate are toxic, extremely inflammable and irritative of the respiratory tract. Furthermore, they pollute the environment.
* The use of these reagents involves extreme care in the preparation process as well as the possibility of contaminating the hydroxytyrosol obtained in this way.

There are several protocols based on the extraction of hydroxytyrosol directly from olive oil, olive leaves or else, from waste waters from the preparation of this oil (Ragazzi and Veronese, 1967; Capasso et al., 1992; 1994; 1999; Monetedoro et al., 1992; Chikamatsu et al., 1996; Capasso et al., 1996; Visioli and Galli, 1998, 1999). Following the above criterion, we are going to disclose the most recent (most optimized) protocols.

Capasso et al. (1999) extract hydroxytyrosol from the waste waters coming from the production of olive oil. The global protocol comprises 4 steps with extraction in ethyl acetate, sodium sulfate, two low pressure chromatographic steps with acetone/petroleum ether phases and finally another step using thin layer chromatography.
* The basic disadvantage, aside from the use of toxic and/or contaminating compounds, is the yield, which is only 1.5% (from 4.2 grams of hydroxytyrosol estimated in the waste waters, 65 mg. are finally obtained). These authors recognize that the protocols for extraction from waste waters are costly and, for the time being, not very feasible for industrial purposes.

The protocol used by Visioli et al. (1999) also extracts hydroxytyrosol from waste waters. This method is patent pending. Nonetheless, the publication discloses the abstract of the protocol by which hydroxytyrosol is isolated in combination with another derivative, and with its precursor, tyrosol. This method comprises 3 chromatographic steps in reverse phase HPLC with the use of ethanol, ethyl acetate and hexane. Although it is not described completely, the disadvantages, a priori, are the same: use of very toxic compounds, several slow steps and a yield that, although not given, can be imagined as being low.

After a combined search of possible patents and scientific articles regarding hydroxytyrosol, up to now, only one patent, in which this molecule is the protagonist, has been found. The patent is: "Hydroxytyrosol as melanin formation inhibitor and lipid peroxide formation inhibitor and its application to topical preparations and bath preparations"; Japanese patent: 8119825, 1996; pp. 1-10. The sources consulted (from 1969 to now) are:
* Data bases: Food Science and Technology Abstract, Medline, Chemical Abstract (CAB) and Agriculture (available for staff members of CSIC (Consejo Superior de Investigaciones Cientificas) at, **ww.cti.csic.es/sis** comu/bbdd).
* Data bases: (aside from the above), BA Biological Abstracts) and CC (Current Contents), available for staff members of ATO in Holland.
* Patent searches: **esp@cenet** (Spanish Patent and Trademark Office; worldwide search); **www.european-patent-office.org** (European Patent Office); **www.uspto.gov** (U.S. Patent and Trademark Office); patent.womplex.ibm.com (IBM Office concerning patents registered in the U.S.A.).

Therefore, two aspects should be emphasized: 1) that there is no method of synthesis other than the chemical method to obtain simple, "natural", non-toxic hydroxytyrosol, without any risk in its obtainment and, 2) hydroxytyrosol has not been proposed as a food additive in order to take advantage of its extraordinary beneficial properties. The lack of a suitable method of obtainment has limited its potential subsequent use.

### 4. DESCRIPTION OF THE INVENTION

### 4.1. Brief description of the invention

After reading the preceding section, it can be inferred that the main inconveniences of the protocols used up to now to obtain hydroxytyrosol are:
* Chemical synthesis, with the subsequent use of highly toxic and contaminating reagents. The protocols are slow and require highly qualified staff members in order to carry them out.
* Furthermore, use of relatively expensive precursors (3,4-dihydroxyphenylacetic acid and oleuropein), aside from the additional cost of the rest of the reagents (solvents, acids, bases, etc.).
* Indispensable use of high resolution chromatography equipment (HPLC) for the purification of the different steps of the protocols.

The method proposed herein achieves the synthesis of the antioxidant hydroxytyrosol from its commercial precursor tyrosol (1994 pesetas per gram, Aldrich), using the enzyme tyrosinase (commercial mushroom enzyme), in the presence of a reducing agent such as Vitamin C (ascorbic acid). The advantages provided are the following:
* The reaction medium occurs at neutral pH, at room temperature and in an aqueous medium.
* No organic solvents, nor dangerous acids nor alkalis, such as sodium hydroxide or hydrochloric acid, nor any toxic or contaminating substance, are used.
* The yield can be 100%; it consists of one or more steps depending on the subsequent use of the product. The protocol is modulable and the required amount of antioxidant can be formed, at a given moment, starting with the same initial conditions.
* Once the optimum conditions of the process are characterized, use of the protocol itself is like a "recipe", easy to use and that does not require any special preparation to carry it out.
* The enzyme is reusable (the enzymes in their catalytic mechanism are not consumed). The reaction rate is linearly dependent on the amount of enzyme used. The reaction is not inhibited by a high concentration of initial precursor (tyrosol) or of the product formed (hydroxytyrosol). The antioxidant can be used alone (isolated with the use of preparative HPLC or TLC techniques) or directly, in combination with vitamin C (strengthened antioxidant action).
* The total cost (estimated calculation) in the production of hydroxytyrosol can be placed, at the maximum, around 5,000 pesetas per gram. The standardized process, and reusing the enzyme, would be even cheaper.
* The process can be used continuously in a bioreactor (already used in many industries), although use thereof is not indispensable.
* The method described herein could be used by any chemical and/or pharmaceutical business firm to obtain and subsequently sell this compound for research.

### 4.2. Detailed description of the invention

The process of enzymatic synthesis of antioxidant hydroxytyrosol is based on o-hydroxylase activity of the enzyme tyrosinase (also called polyphenol oxidase or PPO (Espin et al., 2000b). This enzyme, very abundant in nature and that is found in the entire phylogenetic tree (bacteria, arthropods, birds, mammals, etc.) is commercially available (Sigma, Fluka.... mushroom tyrosinase). PPO can catalyze the inclusion of a hydroxyl group (OH) in "ortho" position in monophenol tyrosol (scheme included herein). Tyrosol lacks antioxidant activity. However, the inclusion of an -OH group in "ortho" position causes a dramatic change in the molecule (Rice-Evans, et al., 1996), giving it a high antioxidant capacity and the rest of the beneficial effects (Visioli and Galli, 1995, 1998; Visioli et al., 1998; Arouma et al., 1998). This same enzyme is also capable of catalyzing oxidation of the new compound formed, hydroxytyrosol, to produce the corresponding yellow o-quinone, which subsequently evolves in order to form melanins. To prevent this undesired subsequent oxidation, vitamin C, which is capable of reducing o-quinone, is added to the reaction medium, instantly reproducing hydroxytyrosol in the medium. Hence, we create an infinite cycle while there is enough vitamin C to block the oxidation process, where the net effect is the accumulation of hydroxytyrosol.

The enzymatic reaction occurs in a medium buffered with phosphate (permitted as a food additive, code E-450) in an aqueous medium, at neutral pH and at room temperature. Hence, the reaction medium consists of: tyrosol, as precursor; (commercial) mushroom tyrosinase to catalyze the process and vitamin C in excess. The reaction starts after initial shaking. The reaction stops when the concentration of the initial tyrosol is exhausted. In order for the reaction to continue, it will suffice to add more tyrosol (always ensuring that the vitamin C/tyrosol ratio is more than 1). The enzyme is not "consumed" in the process since there is no inhibition due to excess substrate or by formation of the product. The reaction rate depends linearly on the concentration of enzyme used. Once the desired concentration of hydroxytyrosol is obtained, (which can be calculated beforehand in a specific amount of time, hence it is a modulable process), the reaction volume (to be chosen, 1 liter, 10 liters, 100 liters, .....) is filtered through a membrane with a pore diameter smaller than 5,000 daltons, or else, it is dialyzed, also with a membrane with a pore diameter smaller than 5,000 daltons. Hence, the filtered volume will contain the low molecular weight components present in the medium (hydroxytyrosol, phosphate and vitamin C with or without tyrosol, depending on how much has been exhausted). The enzyme with a molecular weight twice the size of the pore, is retained in the filter and can be used once again. This first extract, which is very enriched in antioxidant hydroxytyrosol with a high antioxidant capacity (Espin et al., 2000a), (in the present case, in combination with vitamin C), can now be used as a food additive. It should be remembered that vitamin C is an additive (E-300) widely used in the food industry; and hydroxytyrosol, as it has been stated above, is one of the most important phenolic compounds of virgin olive oil, directly responsible for its beneficial effects, its flavor and smell and for the resistance of this oil to become rancid. Thus, its use as a food additive is considered to be interesting. Furthermore, we know that several foreign research groups (mainly Italian) are currently looking for a method like the one described herein that permits rapid and non-contaminating synthesis.

Nonetheless, this first extract can be easily purified by selectively isolating hydroxytyrosol by a single reverse phase preparative chromatography step, HPLC, with a moveable methanol:water phase. It can also be purified with preparative thin layer chromatography, TLC. The compound thus purified would be interesting for the Chemical and/or Pharmaceutical Industry.

Formation of hydroxytyrosol can also be carried out with this same methodology but using waste waters from the production of olive oil. In this case, the global antioxidant capacity of the extract will be enriched with the net formation of o-diphenols from the monophenols present, one of which will be hydroxytyrosol (its monophenol, tyrosol, is very abundant in these waters).

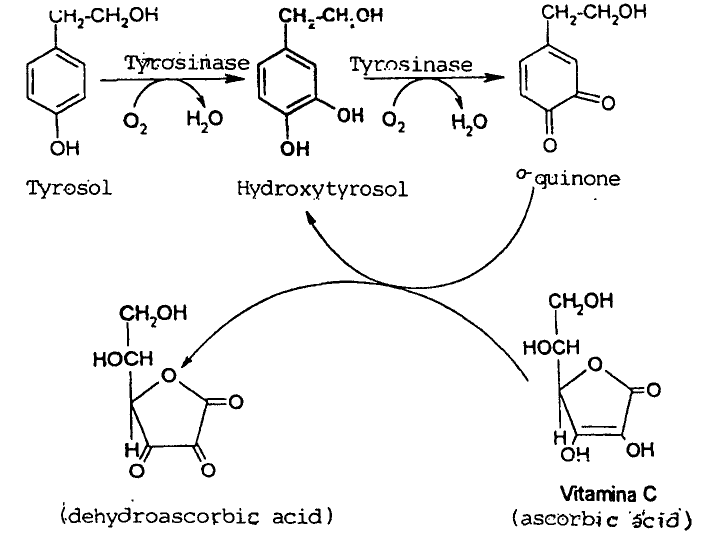

### 5. EMBODIMENT OF THE INVENTION

An initial volume of 1 liter of reaction medium can be used. Obviously this process can be done on an industrial scale, increasing volumes and concentrations. A volume of 1 liter of aqueous solution buffered with phosphate (for example, 25 mM of concentration), and that contains a specific initial concentration of tyrosol that we want to convert into hydroxytyrosol is in a discontinuous or continuous device. For example, we want 1 gram of hydroxytyrosol. Therefore, we add 1 gram of tyrosol (1,944 pesetas) to the medium and 2 grams of vitamin C (30 pesetas). It is homogenized (there are no solubility problems) and commercial mushroom tyrosinase (for example, 15 mg., 4,950 pesetas) is added. The reaction begins. The formation of 1 gram of hydroxytyrosol in approximately 5 hours is estimated under these conditions. If twice the amount of enzyme is added, it will be formed in 2.5 hours. It should be remembered that the enzyme is reusable, so that once an amount of hydroxytyrosol which we consider sufficient (it can be mathematically predicted, or else controlled with an aliquot quantity of the reaction medium and analyzed by HPLC) is formed, more tyrosol can be added to continue forming hydroxytyrosol. When we think that we have formed enough hydroxytyrosol, the volume is filtered or dialyzed and the enzyme can be reused for another process (making it cheaper). The filtered volume can be analyzed by HPLC to corroborate the hydroxytyrosol formed. This volume is lyophilized to obtain its corresponding powder which we will keep frozen.

Hydroxytyrosol can be purified by preparative HPLC or PLC to selectively use it as an additive or else so that it can be commercially distributed. In the HPLC process with a reverse phase column, with a moveable methanol:water phase, ascorbic acid comes out in the front and hydroxytyrosol approximately after 7 minutes. The recollected volume is bubbled with nitrogen, lyophilized and frozen. With preparative TLC, ascorbic acid remains at the application point and hydroxytyrosol moves, clearly separating itself therefrom. We use the same methanol:water phase. Since we know how much it moves (its Rf), we scratch the matrix and then elute with water in order to solubilize the hydroxytyrosol. We bubble in nitrogen, lyophilize and freeze.

In this way, the formation of a gram of hydroxytyrosol initially costs (approximate calculation), 9930 pesetas. However, per each additional gram, in the same process, that will use the same enzyme, it will cost 1944 pesetas for tyrosol and 30 pesetas for vitamin C (1974 pesetas). Therefore, 2 grams will initially cost 8898 pesetas (4449 pesetas/gram); 3 initial grams 10,872 pesetas (3624 pesetas/gram); 10 initial grams 24,690 pesetas (2469 pesetas/gram) and so on. Upon increasing the grams to be transformed, with the same enzyme, the process becomes slower, therefore the process can be balanced out, in the cost/time ratio.

Obviously, these approximate costs, would be drastically reduced if the protocol is used by a chemical or pharmaceutical business firm which would not have to buy the rest of the reaction components, but that would obtain them itself.

Hydroxytyrosol, alone or combined with vitamin C can be used as a food additive in the production of juices (mainly tomato juice), "gazpacho" and other cold soups, baby food, prepared dishes that contain oil, etc. It can be used to stabilize other oils (sunflower seed, soybean, etc.), margarine, butter.

In these processes the effect is multiple:
* Stabilization of the food to which it has been added (it prevents oxidation and the food from becoming rancid).
* It increases the antioxidant capacity of the food with the subsequent increase of nutritional quality.
* The organoleptic characteristics (smell, taste) of the foods can be modified. This additive gives the food an olive oil flavor, or else it will strengthen this flavor if the product already contains this oil.

For the chemical-pharmaceutical industry, the obvious advantage would be that of having a product currently much in demand by many researchers around the world who are obliged to synthesize it and/or extract it themselves.

## Claims

1. Enzymatic synthesis of antioxidant hydroxytyrosol **characterized in that** it comprises the following steps:
* the enzymatic reaction occurs in a medium buffered with phosphate, permitted as a food additive, code E-450, in an aqueous medium, at neutral pH and at room temperature, the reaction medium consisting of: tyrosol, as precursor; commercial mushroom tyrosinase to catalyze the process and vitamin C in excess.
* the reaction starts after initial shaking, the reaction stops when the concentration of the initial tyrosol is exhausted; in order for the reaction to continue, it will suffice to add more tyrosol, -always ensuring that the vitamin C/tyrosol ratio is more than 1 -; the reaction rate depends linearly on the concentration of enzyme used,
* once the desired concentration of hydroxytyrosol is obtained, which can be calculated beforehand in a specific amount of time, hence it is a modulable process, the reaction volume - to be chosen, 1 liter, 10 liters, 100 liters, .....- is filtered through a membrane with a pore diameter smaller than 5,000 daltons, or else, it is dialyzed, also with a membrane with a pore diameter smaller than 5,000 daltons; hence, the filtered volume will contain the low molecular weight components present in the medium - hydroxytyrosol, phosphate and vitamin C with or without tyrosol, depending on how much has been exhausted -; the enzyme with a molecular weight twice the size of the pore, is retained in the filter and can be used once again;
* this first extract, which is very enriched in antioxidant hydroxytyrosol with a high antioxidant capacity can now be used as a food additive.

2. Synthesis according to claim 1, **characterized in that** the first extract obtained is purified by selectively isolating hydroxytyrosol by means of a single chromatography step by reverse phase preparative HPLC, with a moveable methanol:water phase.

3. Synthesis according to claim 1 **characterized in that** the first extract is purified by preparative thin layer chromatography, TLC.

4. Synthesis according to claim 1 **characterized in that** the formation of hydroxytyrosol is carried out by using waste waters from the production of olive oil, enriching the antioxidant capacity of the global extract with the net formation of o-diphenols from the monophenols present, one of which will be hydroxytyrosol.

5. Use of the antioxidant hydroxytyrosol obtained according to the above claims as a food additive to increase the organoleptic and nutritional quality of foods.
